# FASCICULE DE BREVET EUROPEEN

(11) **EP 4 401 787 B1**
(45) Date de publication et mention de la délivrance du brevet: **05.11.2025**
(21) Numéro de dépôt: 22789491.2
(22) Date de dépôt: 13.09.2022
(51) Int. Cl.: A61K 47/54, A61K 47/64, A61P 35/00

(54) **VECTEURS CIBLANT LA BETA-D-N-ACETYLGLUCOSAMINIDASE**
AUF BETA-D-N-ACETYLGLUCOSAMINIDASE GERICHTETE VEKTOREN
VECTORS TARGETING BETA-D-N-ACETYLGLUCOSAMINIDASE

(30) Priorité: 14.09.2021 FR 2109610
(43) Date de publication de la demande: 24.07.2024
(73) Titulaire: Centre National de la Recherche Scientifique, 75016 Paris (FR); Université de Poitiers, 86034 Poitiers Cedex (FR)
(72) Inventeur: PAPOT, Sébastien, 86000 Poitiers (FR); RENOUX, Brigitte, 86340 Nouaille Maupertuis (FR); CHÂTRE, Rémi, 36100 Issoudun (FR)
(74) Mandataire: Novagraaf Technologies
(86) Numéro de dépôt international: PCT/EP2022/075426
(87) Numéro de publication internationale: WO 2023/041531

(56) Documents cités:
- WO-A1-2015/118497
- WO-A1-2019/192979
- RAHUL S. NANDURDIKAR ET AL: "Glycosylated PROLI/NO Derivatives as Nitric Oxide Prodrugs", ORGANIC LETTERS, vol. 12, no. 1, 1 January 2010 (2010-01-01), pages 56 - 59, XP055122344, ISSN: 1523-7060, DOI: 10.1021/ol902481s

## Description

La présente invention a pour objet de nouveaux vecteurs ciblant la bêta-D-N-acétylglucosaminidase, ainsi que les prodrogues correspondantes. Elle a également pour objet lesdits vecteurs et prodrogues pour leur utilisation pour le traitement des cancers.

Le cancer et les maladies inflammatoires sont parmi les affections pathologiques les plus courantes à l'heure actuelle. Parmi les différents modes de traitements envisageables, la chimiothérapie est la seule utilisable à l'encontre des tumeurs circulantes, telles que les lymphomes et les leucémies, et les métastases. Parmi les actifs envisageables en chimiothérapie, figurent certains composés peptidiques naturels à l'image, notamment, de la dolastatine 10, un composé naturel linéaire issu du monde marin, constitué par quatre acides aminés, dont trois lui sont spécifiques. Des dérivés synthétiques de la dolastatine 10 sont aujourd'hui également disponibles et privilégiés. Il s'agit plus particulièrement, de l'auristatine PE, l'auristatine E, ou la monométhyl auristatine E (MMAE). La dolastatine, l'auristatine E et leurs dérivés possèdent la propriété d'inhiber la polymérisation de la tubuline et d'empêcher, de ce fait, la division cellulaire (antimitotiques). Toutefois, ces actifs de la famille des dolastatines, sont malheureusement dénués, à l'image d'autres actifs anticancéreux utilisés cliniquement, d'une sélectivité satisfaisante vis-à-vis des cellules tumorales. En effet, ils ciblent également des tissus sains. Cette destruction non-sélective entraîne de sévères effets secondaires et conduit dans la plupart des cas à un arrêt prématuré du traitement. Le développement de nouveaux agents anticancéreux aptes à détruire sélectivement les tumeurs sans affecter les organes sains représente donc un intérêt majeur dans la lutte contre le cancer.

Le ciblage de la β-glucuronidase avec des vecteurs glucuronylés capables de se lier *in vivo* à l'albumine est une stratégie très efficace pour le traitement d'une grande variété de pathologies malignes, voir par exemple WO2015/118497 et WO2019/192979. Son expression dans le microenvironnement tumoral fait de cette enzyme une cible de choix pour la libération sélective de différents principes actifs. En revanche, dans le but de développer des polychimiothérapies vectorisées, la découverte d'autres enzymes présentes en concentration importante dans la matrice extracellulaire des tumeurs solides est d'un grand intérêt. L'activité de la β-glucuronidase est en effet réduite dans le microenvironnement tumoral en raison du pH qui n'est pas optimal pour son fonctionnement (6 - 6,5). Ainsi, la saturation de cette enzyme, provoquée par l'afflux d'une quantité importante de vecteur dans les tissus malins, pourrait constituer une limite pour cette stratégie de ciblage. Dans ce contexte, le développement de nouvelles gâchettes glycosylées ciblant d'autres enzymes que la β-glucuronidase est une alternative intéressante pour contourner ce problème. En effet, l'utilisation d'un cocktail de vecteurs substrats de différentes enzymes devrait permettre de limiter la saturation de l'activité d'une enzyme unique et ainsi conduire à la libération sélective d'une concentration plus élevée de l'agent anticancéreux dans la tumeur. En outre, cette approche pourrait être adaptée au ciblage de molécules actives différentes dont la libération serait contrôlée par une glycosidase spécifique lors de polychimiothérapies vectorisées, une stratégie encore jamais employée à ce jour.

La β-D-*N*-acétylglucosaminidase est une glycosidase lysosomale présente dans la grande majorité des cellules. Elle possède une activité hydrolytique sélective des résidus *N*-acétylglucosamines présents sur les glycoprotéines qui contribue à l'activation ou l'inactivation des propriétés de ces dernières. La présence de ce motif est issue d'un équilibre entre l'activité de la D-*N*-acétylglucosaminyltransférase, qui fixe les résidus N-acétylglucosamine, et celle de la β-D-*N*-acétylglucosaminidase. De cet équilibre résultent de nombreux processus de signalisations importants pour la prolifération cellulaire. Un déséquilibre de cette homéostasie a été observé dans certains cas de cancer, conduisant à l'apparition de propriétés oncogènes aux protéines (A. Peixoto, M. Relvas-Santos, R. Azevedo, L. L. Santos, J. A. Ferreira, Front. Oncol., 2019, 9, 380 ; H. Nie, H. Ju, J. Fan, X. Shi, Y. Cheng, X. Cang, Z. Zheng, X. Duan, W. Yi, Nat. Commun. 2020, 11, 36 ; R. Muniz de Queiroz, R. Madan, J. Chien, W. B. Dias, C. Slawson, JBC, 2016, 291, 18897 - 18914 ; Z. Ma, K. Vosseller, JBC, 2014, 289, 34457 - 34465 et J. A. Hanover, W. Chen, M. R. Bond, J. Bioenerg. Biomembr., 2018, 50, 155 - 173).

Bien que l'augmentation de la concentration en β-*N*-acétylglucosaminidase soit une caractéristique intéressante pour la libération sélective de principes actifs, très peu de composés substrats de cette enzyme ont été développés à des fins thérapeutiques. R. S. Nandurdikar *et al.* divulguent des prodrogues activées par la N-acétylglucosaminidase pour libérer du monoxyde d'azote (R. S. Nandurdikar, A. E. Maciag, S. Y. Hong, H. Chakrapani, M. L. Citro, L. K. Keefer, J. E. Saavedra, Org. Lett., 2010, 12(1), 56-59).

La présente invention a donc pour but de fournir de nouveaux vecteurs de l'enzyme β-*N*-acétylglucosaminidase.

Ainsi, la présente invention concerne un composé de formule (I) suivante : 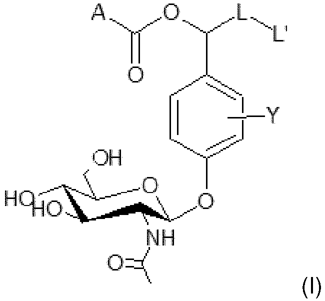 dans laquelle :
- A est un agent anticancéreux,
- Y est un groupe électroattracteur ou électrodonneur,
- L représente un linker répondant à la formule suivante (II) :

   -A₁-A₂-A₃-A₄-A₅-A₆- (II)

   dans laquelle :
   . A₁ représente un radical (C₁-C₆)alkylène, notamment -CH₂-,
   . A₂ représente un groupe obtenu par chimie click, notamment triazole,
   . A₃ représente un radical (C₁-C₆)alkylène, notamment -CH₂-,
   . A₄ représente un radical (C₁-C₃₂)alkylène interrompu par au moins un atome d'oxygène, et étant de préférence un radical polyoxyalkylène, notamment -(CH₂-O-CH₂)₁₀-,
   . A₅ représente un radical (C₁-C₆)alkylène, notamment -CH₂-,
   . A₆ est un radical choisi dans le groupe constitué de : -NR_{c}-, -O- et -S-, R_{c} représentant H ou un groupe (C₁-C₁₂)alkyle, notamment -NH-, et
- L' représente un radical apte à réagir avec une fonction amino, hydroxy ou thiol, et de préférence une fonction thiol,
ainsi que les sels pharmaceutiquement acceptables de celui-ci, ou un mélange racémique, diastéréoisomère ou énantiomère de celui-ci.

Les composés de formule (I) selon l'invention sont des composés vecteurs de l'agent anticancéreux A et substrats de la β-*N*-acétylglucosaminidase.

Les composés de formule (I) peuvent avoir des centres asymétriques. Les composés de la présente invention contenant un atome substitué de manière asymétrique peuvent être isolés sous des formes optiquement actives ou racémiques. Il est bien connu dans le domaine de l'invention comment préparer des formes optiquement actives, comme par résolution de formes racémiques ou par synthèse à partir de matières de départ optiquement actives. Toutes les formes chirales, diastéréoisomères, racémiques et toutes les formes isomères géométriques d'un composé sont visées, à moins que la stéréochimie ou la forme isomère ne soit spécifiquement indiquée.

Le terme « sel pharmaceutiquement acceptable » se réfère à des sels qui conservent l'efficacité biologique et les propriétés des composés de l'invention et qui ne sont pas biologiquement ou autrement indésirables. Dans de nombreux cas, les composés de l'invention sont capables de former des sels d'acide et/ou de base grâce à la présence de groupements amino et/ou carboxyle ou de groupements similaires à ceux-ci. Les sels d'addition d'acides pharmaceutiquement acceptables peuvent être préparés à partir d'acides inorganiques et organiques, tandis que les sels d'addition de bases pharmaceutiquement acceptables peuvent être préparés à partir de bases inorganiques et organiques. Pour une revue des sels pharmaceutiquement acceptables, voir Berge et al. ((1977) J. Pharm. Sd, vol. 66, 1). L'expression « sels pharmaceutiquement acceptables non toxiques » désigne des sels non toxiques formés avec des acides inorganiques ou organiques non toxiques, pharmaceutiquement acceptables ou des bases inorganiques ou organiques. Par exemple, les sels comprennent ceux dérivés d'acides inorganiques tels que les acides chlorhydrique, bromhydrique, sulfurique, sulfamique, phosphorique, nitrique et similaires, ainsi que les sels préparés à partir d'acides organiques tels que acétique, propionique, succinique, glycolique, stéarique, lactique. , malique, tartrique, citrique, ascorbique, pamoïque, maléique, hydroxymaléique, phénylacétique, glutamique, benzoïque, salicylique, sulfanilique, fumarique, méthanesulfonique et toluènesulfonique et similaires.

Comme mentionné ci-dessus, A est un agent anticancéreux.

Selon un mode de réalisation, l'agent anticancéreux est choisi parmi les cytostatiques, les antimétabolites, les substances intercalantes d'ADN, les inhibiteurs de topoisomérase I et II, les inhibiteurs de tubuline, les agents alkylants, la néocarzinostatine, la calichéamycine, la dynemicine ou l'espéramycine A, les inhibiteurs de ribosomes, les inhibiteurs de tyrosine phosphokinase, les composés inducteurs cellulaires différenciation, les inhibiteurs d'histone désacétylase, immunomodulateurs à petites molécules ou les petites molécules ciblant les cellules souches cancéreuses.

Encore plus particulièrement, l'agent anticancéreux selon l'invention est choisi parmi les cytostatiques et les antimétabolites, tels que le 5-fluorouracile, la 5-fluorocytidine, la 5-fluorouridine, la cytosine arabinoside ou le méthotrexate, parmi les substances intercalantes de l'ADN telles que la doxorubicine, la daunomycine, l'idarubicine, l'épirubicine. ou la mitoxantrone, des inhibiteurs de la topoisomérase I et II, tels que la camptothécine, l'étoposide ou le m-AMSA, des inhibiteurs de la tubuline, tels que la vincristine, la vinblastine, la vindésine, le taxol, le nocodazole ou la coléhicine, des agents alkylants, tels que le cyclophosphamide, la mitomycine C, la rachelmycine , cisplatine, gaz moutarde phosphoramide, melphalan, bléomycine, N-bis(2-chloroéthyl)-4-hydroxyaniline, ou de néocarzinostatine, calichéamicine, dynémicine ou espéramycine A, ou d'inhibiteurs de ribosomes, tels que verrucarine A, d'inhibiteurs de tyrosine phosphokinase, tels que la quercétine, la génistéine, l'erbstatine, la tyrphostine ou la rohitukine et leurs dérivés, à partir de composés induisant la différenciation cellulaire, tels que le rétino l'acide ique, l'acide butyrique, les esters de phorbol ou l'aclacinomycine, d'inhibiteurs d'histone désacétylase, tels que CI-994 ou MS275, d'immunomodulateurs, tels que Imiquimod, et de petites molécules ciblant les cellules souches cancéreuses, telles que les inhibiteurs hedgehog comme les dérivés de cyclopamine.

Selon un mode de réalisation, l'agent anticancéreux selon l'invention est choisi parmi les inhibiteurs d'angiogénèse analogues de la combretastatine A, les inhibiteurs de la voie hedgehog tels que la cyclopamine, les inhibiteurs de tyrosine kinase, ou encore les agents immunostimulants.

La famille des dolastatines représente une classe de composés présentant une structure d'au moins 4 acides aminés, dont au moins 3 lui sont spécifiques, c'est-à-dire différents des 20 acides aminés les plus couramment retrouvés dans la nature.

Il pourra être fait référence notamment au document WO 2004/010957, qui décrit des composés conformes à ceux convenant à la présente invention.

Dans un mode de réalisation particulièrement préféré de l'invention A représente un radical dérivant de la dolastatine 10, de l'auristatine PE, de l'auristatine E, de la monométhyl auristatine E et leurs dérivés, de préférence un radical dérivant de la monométhyl auristatine E ou un de ses dérivés.

La différence structurale entre la dolastatine 10 et les composés synthétiques de la sous-famille de l'auristatine réside notamment en la substitution du groupe amino thiazolephenéthyl en position C-terminale de la dolastatine 10, par une unité noréphédrine dans le cas de l'auristatine PE, de l'auristatine E ou de la monométhyle auristatine.

Dans le cadre de la présente invention, et dans un mode de réalisation particulièrement préféré, le radical de la famille des dolastatines est avantageusement choisi parmi la monométhyl auristatine E (MMAE) et un de ses dérivés.

Au sens de l'invention, un dérivé de la dolastatine 10, de l'auristatine PE, de l'auristatine E ou de la monométhyl auristatine E présente une structure chimique très apparentée à au moins l'un de ses actifs et possède des propriétés antimitotiques attribuéé(s) aux composés de la famille des dolastatines.

Sa ou ses différence(s) structurelle(s) peut/peuvent notamment être, par exemple, une substitution sur au moins une chaine latérale d'au moins un des quatre acides aminés qui le compose. Cette substitution peut être réalisée de sorte à contenir ou représenter un groupe alkyle, linéaire, cyclique et/ou ramifié, un groupe aryle, un hétérocycle, un carbocycle.

Cette différence structurelle peut également consister en une modification d'une molécule de la dolostatine 10, de l'auristatine PE ou de l'auristatine E, par exemple au niveau de son amine tertaire en position N-terminale, pour rendre cette fonction compatible avec l'établissement d'une liaison covalente avec le bras de liaison considéré.

Il est des connaissances générales de l'homme du métier de sélectionner les modifications les plus adéquates à ces fins.

Selon un mode de réalisation préféré, A est la monométhyl auristatine E, la doxorubicine ou un dérivé de ceux-ci.

De préférence, A est représenté par la formule (A-1) suivante :

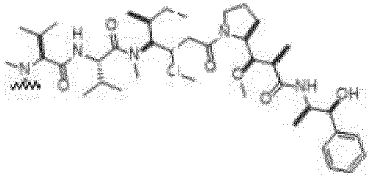

Dans le cadre de la présente invention un *« groupe électroattracteur »* se réfère à la propriété d'un atome ou d'un groupe d'atomes d'attirer les électrons.

On peut par exemple citer les groupes choisis dans le groupe constitué par le groupe NO₂, les esters, le groupe CN, les atomes d'halogène et les groupes alkoxy. De préférence, ledit groupe est choisi dans le groupe constitué par le groupe NO₂, le groupe CO₂Me, le groupe CN, l'atome de fluor, l'atome de brome, l'atome d'iode, l'atome de chlore et le groupe méthoxy.

Dans le cadre de la présente invention un *« groupe électrodonneur »* se réfère à la propriété d'un atome ou d'un groupe d'atomes de donner des électrons.

On entend par groupe électrodonneur, un groupe choisi dans le groupe constitué par le groupe phényle, le groupe hydroxy (OH), un alkyle en C1 à C10, de préférence en C1 à C6, linéaire ou ramifié, un atome d'halogène, un atome d'hydrogène et un groupe alkoxy,
De préférence, Y est un groupe électroattracteur, notamment choisi dans le groupe constitué des atomes d'halogène, de NO₂ et de CF₃. Préférentiellement, Y est NO₂.

Dans les composés de formule (I) selon l'invention, comme indiqué plus haut, le linker L est tel que le groupe A₆ est relié au groupe L' et le groupe A₁ est relié à l'atome de carbone portant le groupe -O-C(=O)-A et le phényle susmentionné.

Selon la présente demande, le terme « alkyle » signifie un groupe hydrocarboné aliphatique saturé ou insaturé qui peut être linéaire ou ramifié ayant, sauf indication contraire, 1 à 12 atomes de carbone dans la chaîne. Les groupes alkyles préférés ont 1 à 6 atomes de carbone dans la chaîne. « Ramifié » signifie qu'un ou des groupes alkyles inférieurs tels que méthyle, éthyle ou propyle sont attachés à une chaîne alkyle linéaire. « Alkyle inférieur » signifie 1 à 4 atomes de carbone dans la chaîne qui peut être droite ou ramifiée.

Le terme « alkylène » tel qu'utilisé ici fait référence à un radical divalent comprenant, sauf indication contraire, de 1 à 6 atomes de carbone. Un radical alkylène correspond à un radical alkyle avec un atome d'hydrogène en moins. Ledit radical lorsqu'il est linéaire peut être notamment représenté par la formule (CH₂)ₙ dans laquelle n est un nombre entier variant de 1 à 6.

De préférence, dans la formule (I), A₁ représente un radical -CH₂-.

Selon l'invention, dans la formule (I), A₂ est un groupe pouvant être obtenu par chimie click. Ce radical est ainsi obtenu par une réaction de chimie click.

Ces réactions de chimie click incluent notamment les cycloadditions de composés insaturés, parmi lesquelles on peut citer les réactions de Diels-Alder entre un diénophile et un diène, et surtout aussi les cycloadditions azoture-alcyne 1,3-dipolaires, et de préférence les cycloadditions catalysées au cuivre cycloaddition azoture-alcyne (CuAAC).

D'autres réactions de chimie click comprennent des réactions impliquant une fonction thiol telles que la formation de thioéthers à partir d'un alcène et de disulfures mixtes, ainsi que des réactions impliquant un groupe carbonyle électrophile de type non aldol, par exemple la formation d'éthers d'oxime à partir d'une oxyamine, d'hydrazones à partir d'une hydrazine ou encore la formation de thiosemicarbazones à partir d'une thiosemicarbazine.

Comme réactions de chimie click, on peut également citer les réactions mettant en jeu des acides thiocarboxyliques ou des thioesters pour conduire à la formation de thioesters et d'amides, ainsi que les réactions entre azotures et phosphines (telles que les ligatures de Staudinger).

De préférence, le radical A₂ est obtenu par réaction entre deux fonctions réactives, ladite réaction étant choisie dans le groupe constitué par :
- la réaction entre un azoture et un alcyne,
- la réaction entre un aldéhyde ou une cétone et un hydrazide,
- la réaction entre un aldéhyde ou une cétone et une oxyamine,
- la réaction entre un azoture et une phosphine,
- la réaction entre un alcène et une tétrazine,
- la réaction entre un isonitrile et une tétrazine, et
- la réaction entre un thiol et un alcène (réaction thiol-ène).

Selon un mode de réalisation préféré, A₂ est un groupe triazole.

De préférence, A₂ est un radical triazole, de préférence un radical répondant à la formule (II-1) suivante :

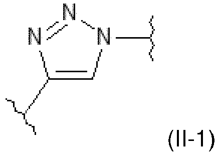

Selon un mode de réalisation, dans la formule (II) susmentionnée, A₃ représente un radical -CH₂-.

Selon un mode de réalisation, dans la formule (II) susmentionnée, A₄ représente un radical polyoxyalkylène

De préférence, A₄ est un groupe de formule -(CH₂-O-CH₂)ₙ-, n étant un nombre entier compris de 1 à 12. En particulier, A₄ est un groupe -(CH₂-O-CH₂)₁₀-.

Selon un mode de réalisation, dans la formule (II) susmentionnée, A₅ représente un radical -CH₂-.

Selon un mode de réalisation, dans la formule (II) susmentionnée, A₆ est un radical -NH-.

Dans la formule (I), comme mentionné ci-dessus, L' est un radical capable de réagir avec une fonction amino, hydroxyle ou thiol.

Dans le cadre de la présente invention, un « radical susceptible de réagir avec une fonction amino, hydroxyle ou thiol » désigne un radical, généralement un radical hydrocarboné, qui possède une fonction, ou unité chimique, capable d'interagir avec un fonction amino secondaire, hydroxyle ou thiol et d'établir ainsi une liaison covalente entre une molécule conjuguée et une entité chimique distincte portant cette fonction compatible avec la réalisation de cette fonction covalente. Dans le cadre de la présente invention, cette entité chimique distincte est plus particulièrement une macromolécule naturellement présente dans un organisme vivant et avantageusement une molécule d'albumine endogène, comme la sérumalbumine humaine.

Selon un mode de réalisation, L' comprend un radical maléimide.

Selon un mode de réalisation, L' répond à la formule suivante :

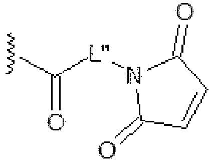

L" représentant un radical (C₁-C₁₂)alkylène, éventuellement substitué par un groupe électroattracteur, notamment un groupe halo(C₁-C₆)alkyle, tel que CF₃, ou un radical phénylène, éventuellement substitué par un groupe électroattracteur, notamment un halogène.

Selon un mode de réalisation préféré, L' est un groupe maléimidocaproyle.

Selon un mode de réalisation, les composés de l'invention répondent à la formule (III) suivante :

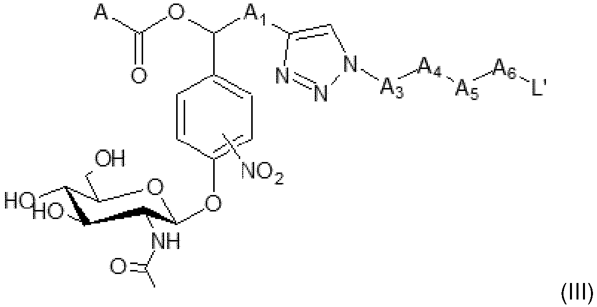

A, A₁, A₃, A₄, A₅, A₆ et L' étant tels que définis ci-dessus.

Selon un mode de réalisation préféré, les composés de l'invention répondent à la formule (IV) suivante : 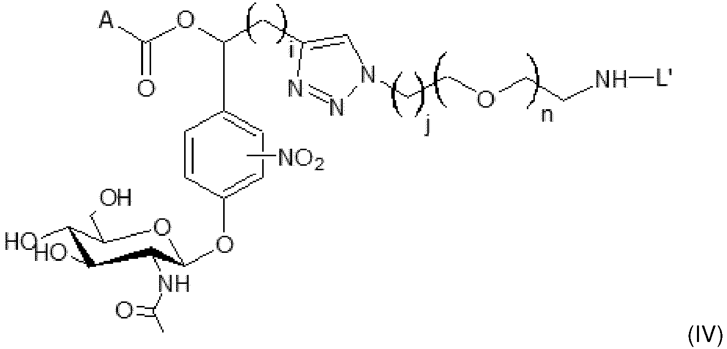
i étant un nombre entier compris de 1 à 6,
j étant un nombre entier compris de 1 à 6,
n étant un nombre entier compris de 1 à 12, et
A et L' étant tels que définis ci-dessus.

Un composé préféré selon l'invention répond à la formule ci-après :

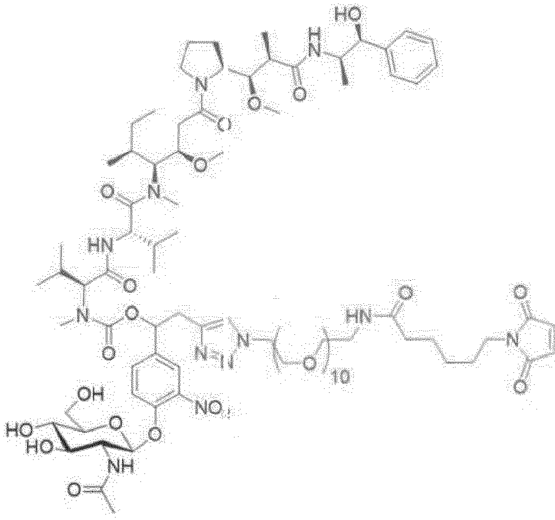

La présente invention concerne également une prodrogue comprenant le composé de formule (I) tel que défini ci-dessus lié par une liaison covalente à une molécule d'albumine ou un de ses fragments ou dérivés.

Au sens de l'invention, le terme « pro-drogue » désigne une molécule apte à véhiculer sous forme inactivée un agent anticancéreux, notamment un composé de la famille des dolastatines, au sein d'un organisme, et de libérer celui-ci dans un organe, un tissu ou des cellules spécifiquement ciblé(e)s, sous l'action d'une β-*N*-acétylglucosaminidase.

Une telle prodrogue répond notamment à la formule (V) suivante :

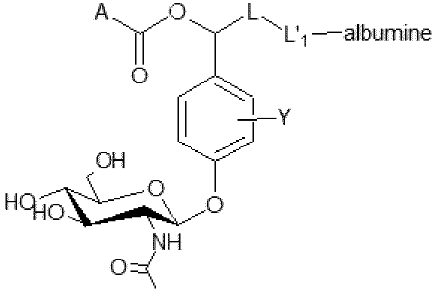

A, L et Y étant tels que définis ci-dessus dans la formule (I).

Le motif L'₁ est quant à lui issu de la réaction entre, d'une part, le radical L' comportant un motif susceptible de réagir avec une fonction libre amino, hydroxyle ou thiol et en particulier avec une fonction thiol libre portée par une macromolécule, avantageusement une molécule d'albumine, encore plus avantageusement de l'albumine sérique.

Dans la présente demande, la prodrogue peut être formée *in vivo* ou *in vitro* avec une macromolécule, de préférence avec une molécule d'albumine.

Ainsi, une albumine endogène ou exogène, et notamment une sérumalbumine humaine, une albumine recombinante ou encore un fragment d'albumine, peut être envisagée.

Selon un mode de réalisation, la liaison covalente entre une molécule du conjugué, telle que décrite par la présente invention, et une molécule d'albumine endogène, notamment une molécule d'albumine sérique humaine, ou un dérivé de celle-ci, est réalisée *in vivo.*

Dans un mode de réalisation, une prodrogue selon l'invention comprend au moins une molécule de conjugué selon l'invention de formule (I) liée par une liaison thioéther au soufre de la cystéine en position 34 d'une molécule d'albumine endogène.

Il a en effet été montré qu'une liaison covalente s'établit spontanément *in vivo* par exemple entre, d'une part, un composé porteur d'un radical capable de réagir avec une fonction thiol et la fonction thiol de la cystéine en position 34 de l'albumine humain (Kratz et al. 2002, J. Med. Chem.).

L'invention concerne également une prodrogue de formule (V) susmentionnée, dans laquelle le groupe -L'₁-albumine répond à la formule (VI) suivante :

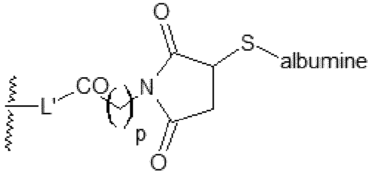

L' étant tel que définis plus haut et p étant un nombre entier compris de 1 à 6, de préférence égal à 5.

Selon un autre mode de réalisation particulier, une prodrogue selon l'invention peut être également formée *in vitro* par au moins une molécule du conjugué liée par une liaison covalente à une molécule d'albumine, une molécule d'albumine recombinante ou un fragment d'une molécule d'albumine ou un de ses dérivés.

Au sens de l'invention il est important que le « fragment d'une molécule d'albumine » désigne un fragment d'une molécule d'albumine présentant une taille suffisante pour garantir une biodisponibilité satisfaisante, une perméabilité vis-à-vis des tissus tumoraux et une imperméabilité vis-à-vis de la barrière endothéliale des tissus sains, de la prodrogue ainsi générée.

Dans ce mode de réalisation particulier, le couplage *in vitro* entre un conjugué de formule générale (I), par son radical L', et une molécule d'albumine, une molécule d'albumine recombinante ou un fragment d'une molécule d'albumine peut être réalisé avec une fonction réactive libre et complémentaire présente au niveau de la molécule d'albumine, la molécule d'albumine recombinante ou le fragment d'une molécule d'albumine.

Dans un mode particulier de réalisation, le fragment d'une molécule d'albumine peut comprendre la cystéine correspondant à la cystéine en position 34 de la séquence de l'albumine endogène.

Contre toute attente, le couplage d'un conjugué de formule générale (I) et d'une molécule d'albumine n'affecte en rien l'aptitude de la prodrogue ainsi formée à :
- être véhiculée et ciblée de manière spécifique dans le microenvironnement du tissu à traiter,
- être clivée dans le microenvironnement du tissu à traiter par une β-glucuronidase, et
- subir, après le clivage du radical N-acétylglucosaminyle, un réarrangement du bras de liaison de sorte à libérer le radical représentant un composé de la famille des dolastatines.

De plus, le couplage entre un conjugué de formule générale (I), par son radical L', et la fonction amino, hydroxy ou thiol d'une molécule d'albumine, en particulier endogène, n'affecte en rien l'aptitude du composé de la famille des dolastatines ainsi libéré, d'exercer son activité biologique, c'est-à-dire son activité antimitotique.

Enfin, le couplage entre un conjugué de formule générale (I), par son radical L', et la fonction amino, hydroxy ou thiol d'une molécule d'albumine, en particulier endogène, limite l'élimination de la prodrogue par les reins. La demi-vie dans le sang d'une prodrogue selon l'invention est ainsi augmentée en comparaison avec celle d'une prodrogue figurée par un composé de la famille des dolastatines fonctionnalisé par un radical N-acétylglucosaminyle.

Dans un autre mode de réalisation de l'invention, la molécule d'albumine, ou fragment d'albumine, de la prodrogue peut être en outre modifié(e), notamment par glycosylation ou par pegylation.

La présente invention concerne également un composé ou conjugué tel que défini ci-dessus de formule (I), ou la prodrogue telle que définie ci-dessus, pour son utilisation comme médicament.

La présente invention concerne également une composition pharmaceutique comprenant un composé tel que défini ci-dessus ou une prodrogue telle que définie ci-dessus, ou un sel pharmaceutiquement acceptable, ainsi qu'au moins un excipient pharmaceutiquement acceptable.

Bien qu'il soit possible d'administrer les composés de l'invention de formule (I) seuls, il est préférable de les présenter sous forme de compositions pharmaceutiques. Les compositions pharmaceutiques, à la fois à usage vétérinaire et à usage humain, utiles selon la présente invention comprennent au moins un composé répondant à la formule (I) telle que définie ci-dessus, conjointement avec un ou plusieurs excipients ou véhicules pharmaceutiquement acceptables et éventuellement d'autres ingrédients thérapeutiques.

Dans certains modes de réalisation préférés, les ingrédients actifs nécessaires à la thérapie combinée peuvent être combinés dans une seule composition pharmaceutique pour une administration simultanée.

Tel qu'utilisé ici, le terme « pharmaceutiquement acceptable » et ses variations grammaticales, lorsqu'ils se réfèrent à des compositions, des supports, des diluants et des réactifs, sont utilisés de manière interchangeable et signifient que les matériaux sont capables d'être administrés à ou sur un mammifère sans production d'effets physiologiques indésirables, tels que nausées, étourdissements, troubles gastriques, etc.

La préparation d'une composition pharmacologique qui contient des ingrédients actifs dissous ou dispersés dans celle-ci est bien comprise dans l'art et n'a pas besoin d'être limitée sur la base de la formulation. Typiquement, ces compositions sont préparées sous forme de produits injectables soit sous forme de solutions liquides ou de suspensions ; cependant, des formes solides appropriées pour une solution, ou des suspensions, dans un liquide avant utilisation peuvent également être préparées. La préparation peut également être émulsionnée. En particulier, les compositions pharmaceutiques peuvent être formulées sous forme galénique solide, par exemple gélules, comprimés, pilules, poudres, dragées ou granulés.

Le choix du véhicule et la teneur en substance active dans le véhicule sont généralement déterminés en fonction de la solubilité et des propriétés chimiques du composé actif, du mode d'administration particulier et des dispositions à respecter en pratique pharmaceutique. Par exemple, des excipients tels que le lactose, le citrate de sodium, le carbonate de calcium, le phosphate dicalcique et des agents délitants tels que l'amidon, les acides alginiques et certains silicates complexes associés à des lubrifiants tels que le stéarate de magnésium, le laurylsulfate de sodium et le talc peuvent être utilisés pour la préparation de comprimés. Pour préparer une capsule, il est avantageux d'utiliser du lactose et des polyéthylèneglycols de haut poids moléculaire. Lorsque des suspensions aqueuses sont utilisées, elles peuvent contenir des agents émulsifiants ou des agents qui facilitent la suspension. Des diluants tels que le saccharose, l'éthanol, le polyéthylèneglycol, le propylèneglycol, le glycérol et le chloroforme ou leurs mélanges peuvent également être utilisés.

Les composés ou conjugués de formule (I), les prodrogues ou les compositions pharmaceutiques selon la présente invention peuvent être administrés par voie orale, parentérale (sous-cutanée, intraveineuse ou intramusculaire) ou localement par application topique sur la peau et les muqueuses.

Les conjugués, prodrogues ou compositions pharmaceutiques conformes à la présente invention peuvent notamment être administrés seuls ou en association avec une chimiothérapie ou une radiothérapie ou encore en association, par exemple, avec d'autres agents thérapeutiques, notamment des agents anticancéreux et antimitotiques, mais également en association avec agents anti-inflammatoires.

Un dosage approprié pour l'invention peut être déterminé selon une approche de routine normalement utilisée dans le domaine de l'invention. L'ajustement de ladite posologie fait clairement partie de la compétence générale de l'homme du métier.

Elle est en effet dépendante, notamment, du poids, de l'âge et du sexe de l'individu à traiter, et de l'état d'évolution de la maladie à traiter.

La présente invention concerne également le composé de formule (I) selon l'invention, ou la prodrogue telle que définie ci-dessus, pour son utilisation pour le traitement et/ou la prévention du cancer.

Également divulguée mais ne relevant pas de l'invention revendiquée est une méthode de traitement d'un cancer comprenant l'administration d'un conjugué de formule générale (I), d'une prodrogue telle que définie ci-dessus, notamment de formule générale (V) ou d'une composition pharmaceutique.

Également divulguée mais ne relevant pas de l'invention revendiquée est une méthode de traitement d'un cancer comprenant l'administration d'un conjugué de formule (I), d'une prodrogue telle que définie ci-dessus, notamment de formule générale (V) ou d'une composition pharmaceutique, en association avec un autre traitement choisi dans un groupe comprenant une chimiothérapie, une radiothérapie, un traitement par au moins un agent anti-inflammatoire et leur combinaison.

Un conjugué ou composé de formule générale (I), une prodrogue selon l'invention, notamment de formule générale (V), ou une composition pharmaceutique selon la présente invention peut être mis en oeuvre, pour son utilisation dans la prévention et/ou le traitement d'un cancer solide, préférentiellement choisi dans un groupe comprenant un neuroblastome, un glioblastome, un ostéosarcome, un rétinoblastome, un sarcome des tissus mous, un cancer du système nerveux central, un néphroblastome, un cancer du poumon, un cancer du sein, un cancer de la prostate, un cancer colorectal, un cancer de la thyroïde, un cancer du col de l'utérus, un cancer de l'endomètre, un cancer des ovaires, un cancer du rein, un cancer du foie, un cancer du cerveau, un cancer des testicules, un cancer du pancréas, un cancer des os, un cancer de la peau, un cancer de l'intestin grêle, un cancer de l'estomac, un cancer de la plèvre, un cancer de l'oesophage, un cancer du larynx et un cancer de la vessie.

Dans un mode de réalisation particulier, le cancer solide est choisi dans le groupe constitué du cancer du pancréas, du cancer des poumons et du cancer du sein.

Dans un mode de réalisation particulier un conjugué de formule générale (I), une prodrogue selon l'invention, notamment de formule générale (V), ou une composition pharmaceutique selon la présente invention peut être mis en oeuvre, pour son utilisation dans la prévention et/ou le traitement de métastases.

### FIGURES

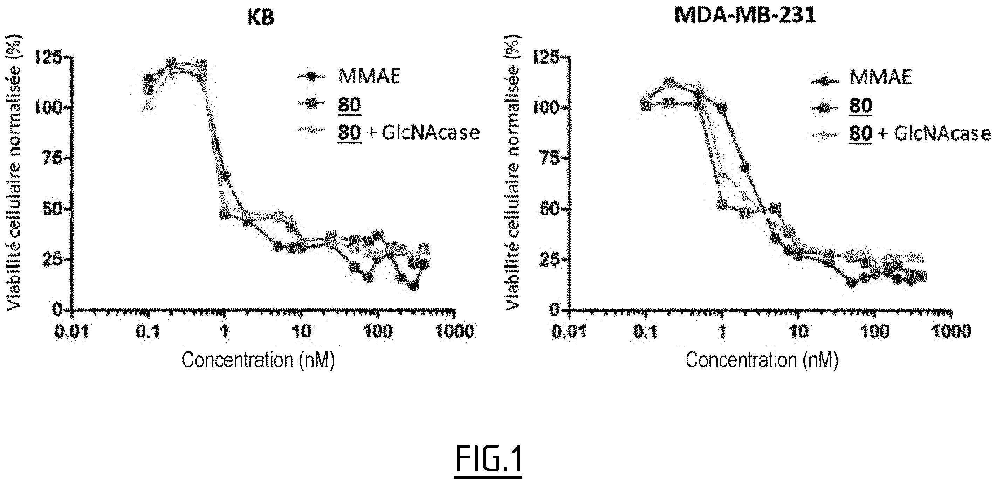

[Fig 1] La Figure 1 représente l'activité antiproliférative de la MMAE et du vecteur 80 (composé selon l'invention) en présence ou absence de β-N-acétylglucosaminidase (GlcNAcase) sur les lignées cellulaires KB et MBA-MB-231 mesurés après 72 heures d'incubation. Les courbes avec les cercles correspondent à la MMAE, les courbes avec les carrés correspondent au vecteur 80 et les courbes avec les triangles correspondent au vecteur 80 en présence de GlcNAcase.

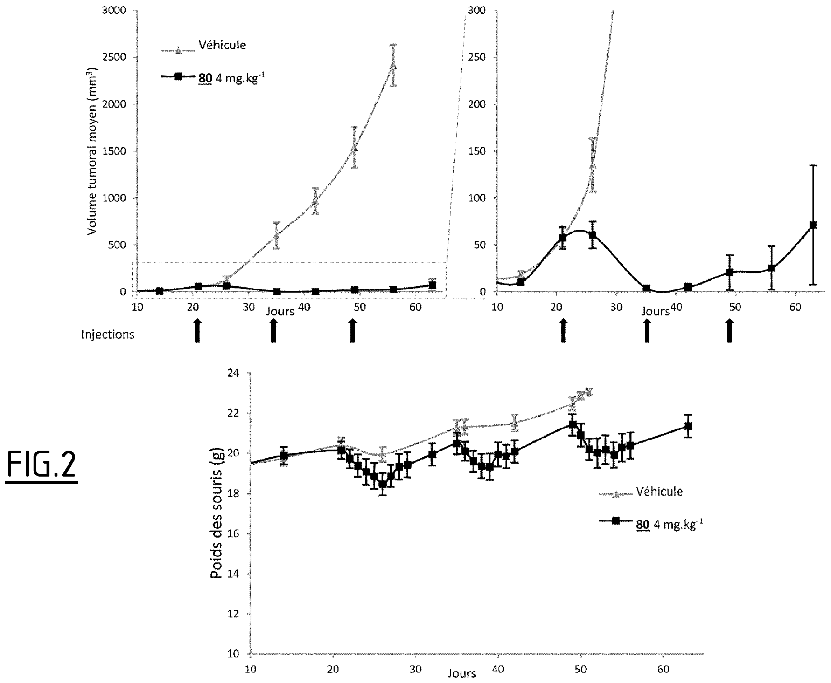

[Fig 2] La Figure 2 représente l'efficacité thérapeutique du vecteur 80 pour le traitement de tumeurs MDA-MB-231 orthotopiques chez la souris (volume initial 58 mm³). Les vecteurs ont été administrés aux jours 21, 35 et 49. Chaque point correspond à la moyenne des volumes tumoraux ± SEM. Les courbes avec les triangles correspondent au véhicule et les courbes avec les carrés correspondent au vecteur 80.

### EXEMPLES

### Réactifs et solvants

Toutes les réactions ont été réalisées sous atmosphère d'argon. Sauf indication contraire, les solvants utilisés étaient de qualité HPLC. Les produits chimiques étaient de qualité analytique provenant de sources commerciales et ont été utilisés sans autre purification.

### Suivi des réactions, purifications et partie analytique

La progression des réactions a été suivie soit par chromatographie liquide, chromatographie liquide couplée à la spectroscopie de masse ou sur des plaques de CCM en gel de silice pré-enduites MACHEREY-NAGEL ALUGRAM^{®} SIL G/UV254 (0,2 mm de gel de silice 60). Les taches ont été visualisées sous une lumière UV à 254 nm et/ou en immergeant la plaque CCM dans une solution d'acide phosphomolybdique (3 g) dans de l'éthanol (100 ml) suivi d'un chauffage avec un pistolet thermique.

Les chromatographies automatiques ont été réalisées avec un instrument COMBIFLASH^{®} RF 200I TELEDYNE ISCO équipé de détecteur UV et ESLD et utilisant des cartouches flash de silice Interchim^{®} 15 ou 50 µm pour la chromatographie en phase normale et HP C18 RediSep^{®} GOLD 4g ou 15,5g pour la chromatographie en phase inverse.

Les spectres RMN ¹H et ¹³C ont été enregistrés à 400 MHz et 100 MHz respectivement sur un instrument Bruker 400 Avance III, équipé d'un aimant ultra-blindé et d'une sonde large bande BBFO 5 mm. Pour les composés sélectionnés, les RMN ¹H et ¹³C ont été enregistrées à 500 MHz et 126 MHz respectivement sur un spectromètre Bruker équipé d'une cryosonde TXI ¹H-¹³C-¹⁵N (5mm) dans la plateforme Prism de l'Université de Rennes. Les déplacements chimiques (δ) sont rapportés en parties par million (ppm) de champ faible à élevé et référencés au solvant résiduel. Les constantes de couplage (J) sont exprimées en hertz (Hz).

La masse exacte a été déterminée pour tous les dérivés par leur infusion sur des spectromètres de masse ESI haute résolution au CBM/ICOA FR2708, à l'Université d'Orléans et au Centre d'Analyse Organique de l'IC2MP à l'Université de Poitiers.

La RP-HPLC analytique a été réalisée sur un système Dionex Ultimate 3000 équipé d'un détecteur UV/Visible à longueur d'onde variable avec une colonne chromatographique en phase inverse MACHEREY-NAGEL NUCLEOSHELL^{®} (150/4,6, RP18, 5 µm) à 30°C et 1 mL.min-¹. La méthode 1 a utilisé un gradient linéaire composé de A (0,2 % de TFA dans l'eau) et de B (CH₃CN) commençant par 20 % de B et atteignant 100 % de B en 30 min. Tous les chromatogrammes ont été enregistrés à 254 nm.

La LC-MS analytique a été réalisée sur un appareil Shimadzu LCMS-2020. Une colonne chromatographique en phase inverse MACHEREY-NAGEL NUCLEOSHELL^{®} (150/4,6, RP18, 5 pm) à 40°C a été utilisée pour la séparation chromatographique à un débit de 1 ml.min-¹. L'effluent de la colonne a été introduit dans la source d'ionisation électrospray (ESI) du spectromètre de masse. Les analyses ont été effectuées en mode ion positif et négatif. La tension d'électrospray a été fixée à 4,5 kV. Les températures du capillaire et du réchauffeur étaient de 250°C et 400°C respectivement. Les débits de gaz de séchage (azote) et de gaz de nébulisation (azote) ont été fixés respectivement à 15 L.min-¹ et 1,5 L.min-¹. L'analyse des données a été réalisée avec le logiciel LabSolutions. Les expériences LC/MS ont été réalisées en utilisant un gradient linéaire composé de A (0,1 % d'acide formique dans l'eau) et B (0,1 % d'acide formique dans CH₃CN) commençant avec 20 % de B et atteignant 100 % de B en 15 min (Méthode 2).

### Modes opératoires

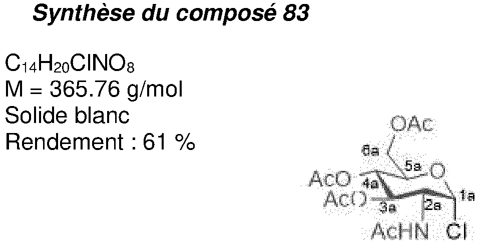

La N-acétylglucosamine (3,0 g, 13,5 mmol, 1 équiv.) a été solubilisée dans du chlorure d'acétyle (13,5 ml, 190 mmol, 14 équiv.) et la solution a été agitée pendant 72 heures à température ambiante. Après achèvement, la solution a été hydrolysée par addition d'eau glacée (150 ml), agitée pendant 5 minutes et du CH₂Cl₂ (60 ml) a été ajouté. La phase organique a été séparée, lavée avec du NaHCO₃ saturé (2 x 60 ml) et de la saumure (60 ml). Les phases organiques combinées ont été séchées sur MgSO₄, filtrées et concentrées sous vide. Le résidu brut a été purifié par chromatographie sur une colonne de gel de silice (EP/AcOEt 50/50 à 0/100 en 20 minutes) pour donner le composé **83** (3,0 g, 61 %) sous la forme d'un solide blanc.

**R_{f}:** 0,37 (EP/AcOEt 40/60)
**MNR¹H (400 MHz, CDCl₃, 298K):** δ ppm = 6.19 (d, *J =* 3.7 Hz, 1H, H1a), 5.77 (d, *J =* 9.0 Hz, 1H, HNH), 5.32 (dd, *J* = 14.6, 5.5 Hz, 1H), 5.22 (t, *J =* 9.7 Hz, 1H), 4.53 (ddd, *J* = 10.6, 8.8, 3.8 Hz, 1H, H2a), 4.37 - 4,23 (m, 2H), 4.14 (dd, *J* = 13.1, 2.7 Hz, 1H), 2.11 (s, 3H), 2.06 (s, 3H), 2.06 (s, 3H), 1.99 (s, 3H).

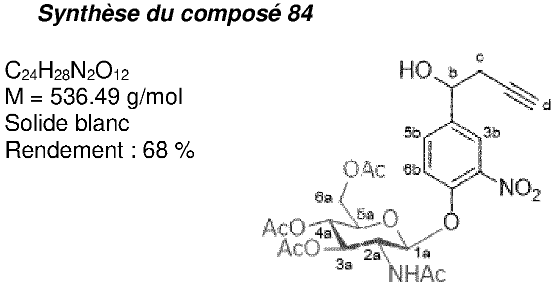

A une solution de phénol 55 (cf. WO2011/145068)(169 mg, 0,82 mmol, 1,5 équiv.) solubilisé dans un mélange de CH₂Cl₂ (2 mL) et de NaHCO₃ aqueux (1 M, 1,4 mL) a été ajouté de l'iodure de tétrabutylammonium (176 mg, 0,54 mmol, 1 équiv.). Le mélange a été agité à température ambiante pendant 15 minutes. Ensuite, une solution du composé **83** (200 mg, 0,54 mmol, 1 équiv.) dans du CH₂Cl₂ (1 mL) a été ajoutée et le mélange a été agité jusqu'à consommation totale du composé chloré **83.** Après 5h30, la phase organique a été séparée, lavée avec de l'eau (5 ml), de la saumure (5 ml), séchée sur MgSO₄, filtrée et concentrée sous vide. Le résidu brut a été purifié par chromatographie sur une colonne de gel de silice (EP/AcOEt 100/0 à 0/100 en 20 minutes) pour donner le composé **84** sous la forme d'un mélange de 2 diastéréoisomères (200 mg, solide blanc, 68 %).

**R_{f}:** 0,31 (CH₂Cl₂/MeOH 95/5)
**RMN ¹H (500 MHz, CDCl₃, 298K):** δ ppm = 7.85 (2d, *J* = 2.1 Hz, 1H, H_{3b}), 7.62 - 7.43 (m, 1H, H_{5b}), 7.36 (d, *J* = 8.6 Hz, 1H, H_{6b}), 5.77 (d, *J* = 8.2 Hz, 1H, H_{NH}), 5.59 (dd, *J* = 10.4, 9.1 Hz, 1H, H₃ₐ), 5.50 (dd, *J* = 8.2, 1.1 Hz, 1H, H₁ₐ), 5.13 (t, *J* = 9.5 Hz, 1H, H₄ₐ), 4.97 - 4.84 (m, 1H, He), 4.34 - 4.15 (m, 2H, H₆ₐ), 3.97 - 3.81 (m, 2H, H₂ₐ and H₅ₐ), 2.73 - 2.57 (m, 2H, H_{c}), 2.11 (t, *J* = 2.6 Hz, 1H, H_{d}), 2.09 (s, 3H, H_{acetate}), 2.06 (s, 3H, H_{acetate}), 2.05 (s, 3H, H_{acetate}), 1.99 (s, 3H, H_{acetamide}).

**RMN** ¹³C **(126 MHz, CDCl₃, 298K):** δ ppm = 171.28, 170.71, 170.61, 169.63, 148.86, 138.91, 131.29, 122.65, 121.20, 121.14, 99.71, 79.53, 77.41, 77.16, 76.91, 72.37, 72.20, 71.28, 70.83, 68.59, 62.04, 55.48, 29.62, 23.50, 20.91, 20.85, 20.81.

**HRMS (ESI):** [M+Na]⁺ calculé pour C₂₄H₂₈N₂NaO₁₂ : 559.1534 trouvé 559.1521.

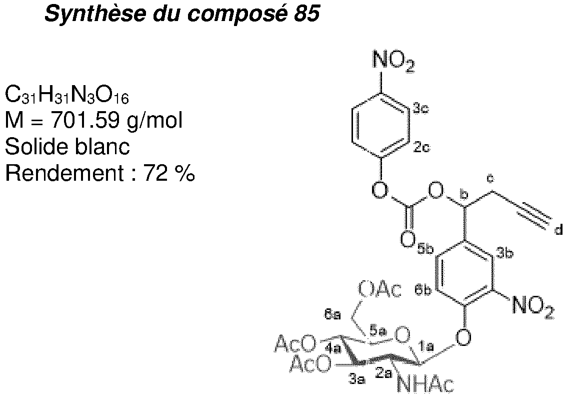

A une solution de phénol **84** (200 mg, 0,37 mmol, 1 équiv.) solubilisé dans du CH₂Cl₂ (4 ml) a été ajouté du chloroformiate de 4-nitrophényle (150 mg, 0,75 mmol, 2 équiv.) à température ambiante. Le mélange a été refroidi à 0°C et de la pyridine (75,2 µl, 0,93 mmol, 2,5 équiv.) a été ajoutée. Le mélange a été agité à 0°C pendant 20 minutes et laissé se réchauffer à température ambiante pendant 3 heures. Après achèvement, le mélange a été hydrolyse avec du NaHCO₃ saturé (4 ml) et agité pendant 5 minutes. Ensuite, la phase organique a été séparée et la phase aqueuse a été extraite avec du CH₂Cl₂ (4 ml). Les phases organiques combinées ont été lavées avec de la saumure (5 ml), séchées sur MgSO₄, filtrées et concentrées sous vide. Le résidu brut a été purifié par chromatographie sur une colonne de gel de silice (CH₂Cl₂/MeOH 100/0 à 95/5 en 30 minutes) pour donner le composé **85** sous la forme d'un mélange de 2 diastéréoisomères (188 mg, solide blanc, 72 %).

**R_{f}:** 0,17 (CH₂Cl₂/MeOH 98/2)
**RMN ¹H (500 MHz, CDCl₃, 298K):** δ ppm = 8.28 (d, *J* = 9.2 Hz, 2H, H_{3c}), 7.91 (t, *J* = 2.2 Hz, 1H, H_{3b}), 7.62 (dt, *J* = 8.6, 2.1 Hz, 1H, H_{5b}), 7.43 - 7.30 (m, 3H, H_{6b} and H_{2c}), 5.91 - 5.72 (m, 2H, H_{b} and H_{NH}), 5.72 - 5.54 (m, 2H, H₃ₐ and H₁ₐ), 5.13 (t, *J* = 9.5 Hz, 1H, H₄ₐ), 4.35 - 4.17 (m, 2H, H₆ₐ), 3.99 - 3.88 (m, 1H, H₅ₐ), 3.88 - 3.79 (m, 1H, H₂ₐ), 3.01 - 2.78 (m, 2H, H_{c}), 2.12 - 2.08 (m, 4H, H_{d} and H_{acetate}), 2.07 (s, 3H, H_{acetate}), 2.05 (s, 3H, H_{acetate}), 1.98 (s, 3H, H_{acetamide}).

**RMN** ¹³C **(126 MHz, CDCl₃, 298K):** δ ppm = 171.35, 171.31, 170.66, 170.52, 169.61, 155.27, 151.62, 149.92, 145.70, 141.44, 141.30, 133.46, 133.41, 132.46, 132.26, 125.52, 123.76, 123.57, 121.85, 121.83, 120.79, 120.65, 99.31, 99.18, 72.61, 72.41, 71.02, 70.98, 68.54, 61.99, 60.56, 55.69, 55.62, 26.37, 23.50, 21.22, 20.89, 20.83, 20.80, 14.33.

**HRMS (ESI):** [M+Na]⁺ calculé pour C₃₁H₃₁N₃NaO₁₆ : 724.1597 trouvé 724.1604.

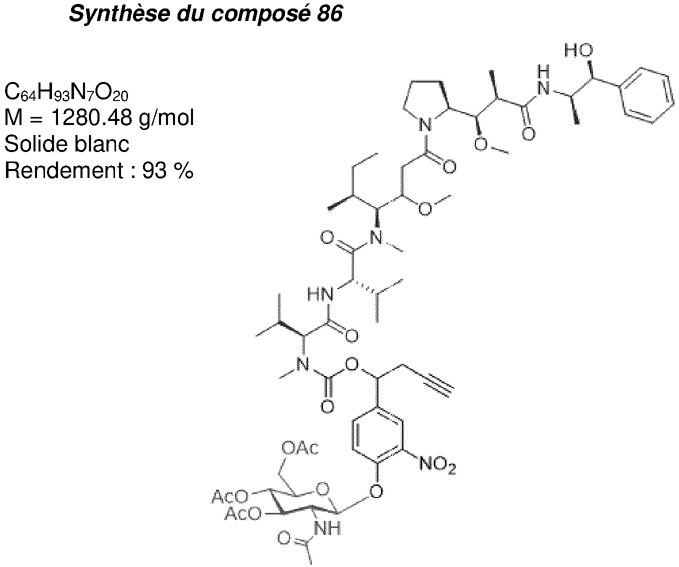

A une solution de carbonate **85** (98 mg, 0,139 mmol, 1 équiv.) solubilisé dans du DMF anhydre (3 mL) ont été ajoutés de la MMAE (100 mg, 0,139 mmol, 1 équiv.) et du HOBt (19 mg, 0,139 mmol, 1 équiv.). Le mélange a été agité à température ambiante et de la pyridine (0,7 ml) a été ajoutée. Le mélange a été agité à température ambiante pendant 24 heures. Après achèvement, les solvants ont été évaporés sous pression réduite et le résidu brut a été purifié par chromatographie sur colonne de gel de silice (CH₂Cl₂/MeOH 100/0 à 90/10 en 30 minutes) pour donner le composé **86** sous la forme d'un mélange de 2 diastéréoisomères (166 mg, solide blanc, 93 %).

**Rt** = 10.20 min (Method 2)
**MS (ESI):** [M+H]⁺ calculé pour C₆₄H₉ₐN₇O₂₀ : 1280.6 trouvé 1280.9 ; [M+2H]²⁺ calculé pour C₆₄H₉₅N₇O₂₀ : 640.8 trouvé 641.3.

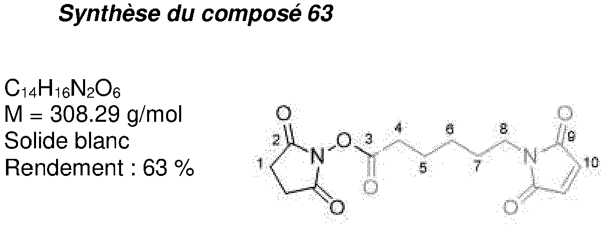

De l'anhydride maléique (800 mg, 8,16 mmol, 1 équiv.) a été ajouté à une solution d'acide 6-aminohexanoïque (1,07 g, 8,16 mmol, 1 équiv.) dans du DMF (10 ml) et agité à température ambiante. Après 2 heures, le mélange a été refroidi à 0°C, du *N*-hydroxysuccinimide (1,13 g, 9,79 mmol, 1,2 équiv.) et EDC.HCl (3,91 g, 20,4 mmol, 2,5 équiv.) ont été ajoutés, puis chauffés jusqu'à 35 °C pendant 12 heures. Après refroidissement à température ambiante, le mélange a été dilué avec du CH₂Cl₂ (200 ml), lavé avec du NaHCO₃ saturé, de l'eau et de la saumure. La phase organique a été séchée sur MgSO₄, filtrée et concentrée sous pression réduite. Le produit brut a été purifié par chromatographie sur colonne sur gel de silice (AcOEt/EP 60/40) pour donner le composé **63** (1,53 mg, 63 %) sous la forme d'un solide blanc.

**Rf** : 0,41 (AcOEt/EP 60/40)
**RMN** ¹H **(400 MHz, CDCl₃, δ ppm):** 6.67 (s, 2H, H₁₀), 3.50 (t, 2H, *J* = 7.17 Hz, H₈), 2.81 (sl, 4H, H₁), 2.58 (t, 2H, *J* = 7.40 Hz, H₄), 1.75 (m, 2H, H₇), 1.61 (m, 2H, H₅), 1.39 (m, 2H, H₆).

**RMN ¹³C (75 MHz, CDCl₃, δ ppm):** 170.8 (C₉), 169.1 (C₂), 168.3 (C₃), 134.0 (C₁₀), 37.4 (C₈), 30.8 (C₄), 28.0 (C₇), 25.8 (C₆), 25.6 (C₁), 24.1 (C₅).

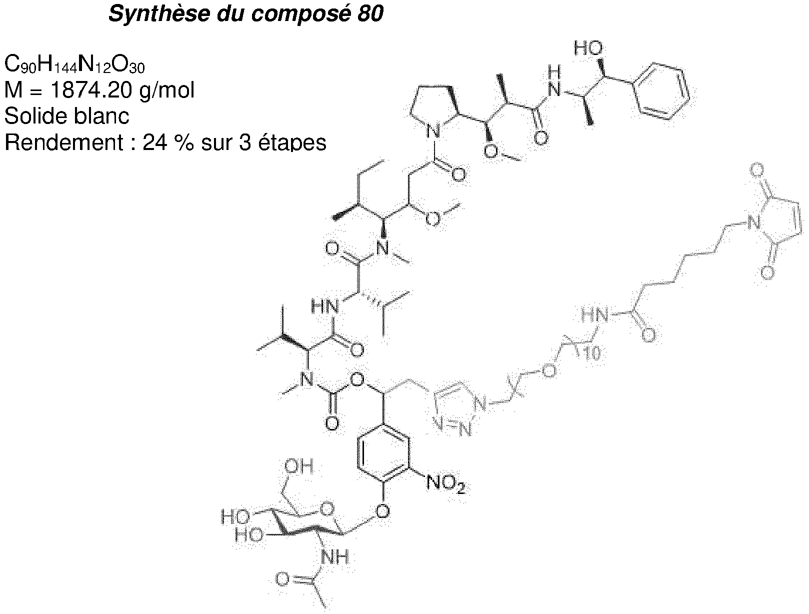

Le vecteur **80** a été préparé à partir du composé **86** en 3 étapes sans purifications intermédiaires.

Tout d'abord, à une solution d'alcyne **86** (70 mg, 0,055 mmol, 1 équiv.) et d'azido-PEG10-amine **46** (32 mg, 0,060 mmol, 1,1 équiv.) dans du CH₂Cl₂ dégazé (2 mL) sous atmosphère d'argon a été ajouté du Cu(MeCN)₄PF₆ (20 mg, 0,055 mmol, 1 équiv.). Le mélange a été agité à température ambiante jusqu'à achèvement. Après 3 heures, la résine QuadraPure^{®} IDA (300 mg) a été ajoutée au mélange pour piéger le cuivre. La solution a été agitée pendant 3 heures supplémentaires et la résine retirée par filtration. Le solvant a été évaporé sous pression réduite et le brut a été utilisé immédiatement pour l'étape suivante sans autre purification.

Deuxièmement, le composé brut **87** a été solubilisé dans du MeOH (2 ml). Ensuite, du méthanolate de sodium (0,44 mg, 0,008 mmol, 0,15 équiv.) a été ajouté et le mélange a été agité à température ambiante pendant 5 heures. Après achèvement, la résine IR-120 (300 mg) a été ajoutée jusqu'à pH neutre. Le solvant a été évaporé sous pression réduite. Le brut a été utilisé immédiatement pour l'étape suivante sans autre purification.

Enfin, de la triéthylamine (23 µL, 0,164 mmol, 3 équiv.) a été ajouté à une solution de l'amine brute **88** et de l'ester NHS **63** (18,5 mg, 0,060 mmol, 1,1 équiv.) dans du DMSO anhydre (2 mL). Le mélange a été agité à température ambiante pendant 1 heure. Après achèvement, contrôlé par LC-MS (Méthode 2), le solvant a été évaporé sous pression réduite et le résidu brut a été purifié par chromatographie en phase inverse sur silice greffée C18 (gradient d'élution MeCN/H₂O (0,05 % TFA) 20/80 à 80/ 20 sur 30 minutes) pour donner le vecteur **80** sous la forme d'un mélange de 2 diastéréoisomères (25 mg, solide blanc, 24 % sur 3 étapes).

**Rt** = 7.33 min (Method 2)
**HRMS (ESI):** [M+Na]⁺ calculé pour C₉₀H₁₄₄N₁₂NaO₃₀ : 1896.0004 trouvé 1895.9952.

### EVALUATION BIOLOGIQUE DU VECTEUR 80

### Evaluation de l'activité antiproliférative du vecteur 80

L'activité antiproliférative du vecteur 80 a été évaluée sur les lignées tumorales humaines KB et MDA-MB-231 et comparée à celle de la MMAE. Pour cela, le composé 80 a été placé dans le milieu de culture en absence ou en présence de β-N-acétylglucosaminidase et la viabilité cellulaire a été mesurée après 72 heures d'incubation (Figure 1).

On constate que le vecteur 80 possède une activité antiproliférative similaire à celle de la MMAE. En effet, son incubation en absence ou en présence de β-N-acétylglucosaminidase conduit à la cytotoxicité identique, sur les deux lignées cellulaires.

Ce résultat surprenant montre que le vecteur 80 ne permet pas de masquer la toxicité de la MMAE, contrairement à son analogue glucuronylé 24. Ce résultat pourrait être expliqué par la différence de polarité entre les glucuronides et les N-acétylglucosaminides qui ne présentent pas de fonctions chimiques ionisables (acide carboxylique). Ainsi, le composé 80 pourrait pénétrer passivement au travers de la membrane cellulaire, puis être activé par la β-*N*-acétylglucosaminidase lysosomale pour conduire à la libération de la MMAE. Une seconde hypothèse est basée sur la libération de la β-*N*-acétylglucosaminidase par ces cellules cancéreuses. En effet, il a été démontré que ces dernières pouvaient sécréter cette enzyme dans le milieu de culture. Ce phénomène pourrait alors être responsable de l'activation du vecteur 80 indépendamment de l'addition de la β-*N*-acétylglucosaminidase au milieu de culture.

### Evaluation de l'efficacité thérapeutique de la prodrogue 80 in vivo

L'efficacité thérapeutique du vecteur 80 a été évaluée chez la souris, sur un modèle de tumeur mammaire triple négatif de type MDA-MB-231 (Figure 2). Le composé 80 a été administré trois fois, avec un délai de 14 jours, par voie intraveineuse dans la veine caudale, à une dose de 4 mg.kg⁻¹.

On constate que le vecteur 80 possède une activité thérapeutique significative par rapport au groupe contrôle. En effet, une réduction de la masse tumorale est obtenue, dès la première administration. De plus, à l'issue de la première injection, 4/6 souris traitées par le vecteur 80 ne présentaient plus de tumeurs détectables à partir du jour 35.

Le vecteur 80 a également été bien supporté par les animaux puisqu'aucune mortalité n'a été observée jusqu'à la fin du protocole (jour 63). Au jour 56, les traitements avec le vecteur 80 ont conduit à une forte inhibition de la croissance tumorale par rapport au groupe contrôle (99 %). A la fin de l'expérience (jour 63), une régression totale et durable de la tumeur a été obtenue pour 3/6 souris. En revanche, une reprise de la tumorigénèse a été observée pour une des souris qui ne présentait plus de tumeur détectable au jour 35.

La réponse initiale au traitement avec 80 est également très importante, conduisant à une réduction de 90 % de la taille initiale des tumeurs. Cependant, au jour 42 du protocole, une reprise de la croissance tumorale a été constatée. Il semblerait également que l'administration suivante (jour 49) ait relativement peu d'effet,

### Conclusion

Le but était d'étudier le ciblage de la β-D-*N*-acétylglucosaminidase afin de libérer sélectivement un agent cytotoxique au niveau des tumeurs. Dans ce cadre la synthèse et l'évaluation biologique du vecteur N-acétylglucosaminylé de la MMAE 80 ont été réalisées. Ce dernier comporte une N-acétylglucosamine reliée à la MMAE par un espaceur auto-immolable. Le ciblage des tissus cancéreux est rendu possible par la présence du groupement maléimide de 80 pouvant réagir *in vivo* avec la fonction thiol de l'albumine plasmatique par une réaction d'addition de Michael.

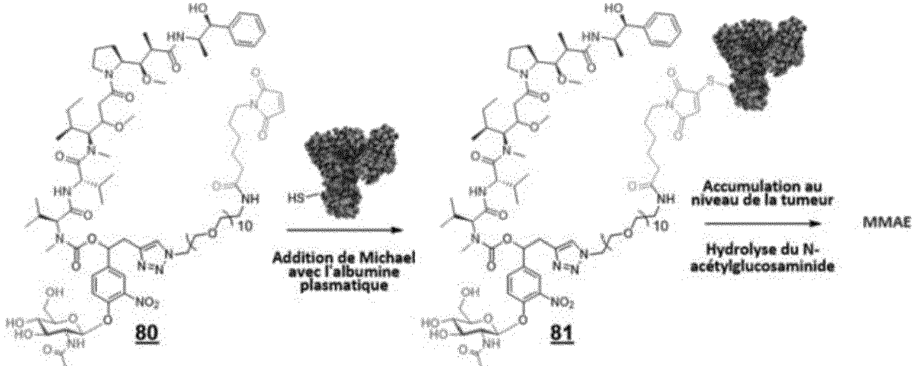

Le vecteur macromoléculaire 81 formé est alors capable de s'accumuler au niveau de la tumeur grâce au tropisme physiopathologique de l'albumine au niveau de la malignité ainsi qu'aux défauts de vascularisation liés à la néo-angiogenèse tumorale par effet EPR.

L'efficacité thérapeutique de ce nouveau vecteur a été évaluée sur un modèle de tumeur mammaire triple négative MBA-MB-231 implantées chez la souris. Ces essais ont montré que le vecteur 80 présente une activité anticancéreuse très importante. Ainsi, le développement de vecteurs N-acétylglucosaminylés est une approche très prometteuse pour le développement de nouveaux traitements sélectifs et efficaces des pathologies malignes.

## Revendications

1. Composé de formule (I) suivante : 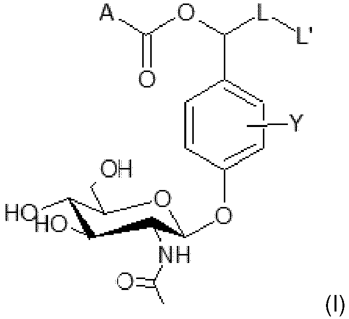 dans laquelle :
- A est un agent anticancéreux,
- Y est un groupe électroattracteur ou électrodonneur,
- L représente un linker répondant à la formule suivante (II) :
-A₁-A₂-A₃-A₄-A₅-A₆- (II)
dans laquelle :
. A₁ représente un radical (C₁-C₆)alkylène,
. A₂ représente un groupe obtenu par chimie click,
. A₃ représente un radical (C₁-C₆)alkylène,
. A₄ représente un radical (C₁-C₃₂)alkylène interrompu par au moins un atome d'oxygène, et étant de préférence un radical polyoxyalkylène,
. A₅ représente un radical (C₁-C₆)alkylène,
. A₆ est un radical choisi dans le groupe constitué de : -NR_{c}-, -O- et -S-, R_{c} représentant H ou un groupe (C₁-C₁₂)alkyle, et
- L' représente un radical apte à réagir avec une fonction amino, hydroxy ou thiol, et de préférence une fonction thiol,
ainsi que les sels pharmaceutiquement acceptables de celui-ci, ou un mélange racémique, diastéréoisomère ou énantiomère de celui-ci.

2. Composé selon la revendication 1, dans lequel A₂ est un groupe triazole.

3. Composé selon la revendication 1 ou 2, répondant à la formule (III) **suivante :** 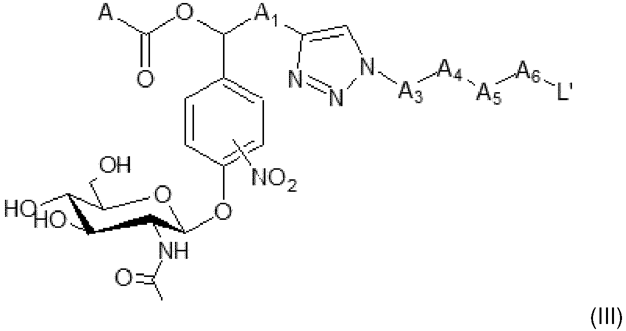 A, A₁, A₃, A₄, A₅, A₆ et L' étant tels que définis dans la revendication 1.

4. Composé selon l'une quelconque des revendications 1 à 3, dans lequel A₄ est un groupe de formule -(CH₂-O-CH₂)ₙ-, n étant un nombre entier compris de 1 à 12.

5. Composé selon l'une quelconque des revendications 1 à 4, répondant à la formule (IV) suivante : 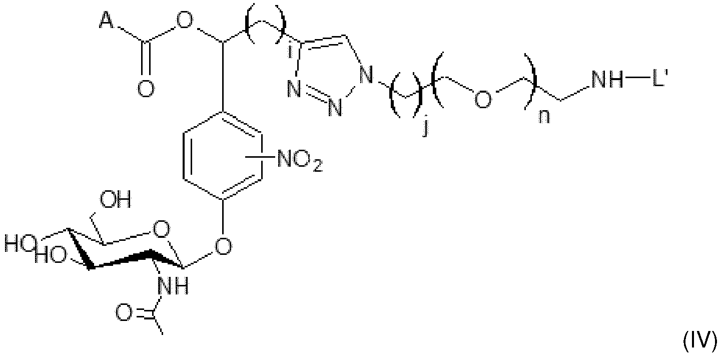
i étant un nombre entier compris de 1 à 6,
j étant un nombre entier compris de 1 à 6,
n étant un nombre entier compris de 1 à 12, et
A et L' étant tels que définis dans la revendication 1.

6. Composé selon l'une quelconque des revendications 1 à 5, dans lequel L' est un groupe maléimidocaproyle.

7. Composé selon l'une quelconque des revendications 1 à 6, dans lequel A **est la** monométhyl auristatine E, la doxorubicine ou un dérivé de ceux-ci.

8. Composé selon l'une quelconque des revendications 1 à 7, répondant à la formule suivante :

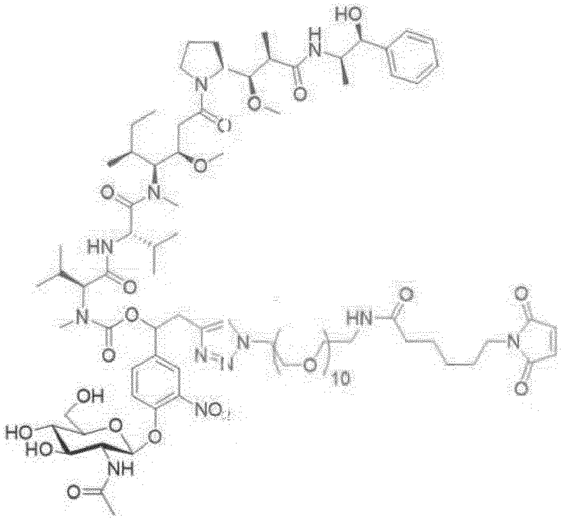

9. Prodrogue comprenant le composé selon l'une des revendications 1 à 8 lié par une liaison covalente à une molécule d'albumine ou un de ses fragments ou dérivés.

10. Composé selon l'une quelconque des revendications 1 à 8, ou la prodrogue selon la revendication 9, pour son utilisation comme médicament.

11. Composition pharmaceutique comprenant un composé selon l'une quelconque des revendications 1 à 8 ou une prodrogue selon la revendication 9, ou un sel pharmaceutiquement acceptable, ainsi qu'au moins un excipient pharmaceutiquement acceptable.

12. Composé selon l'une quelconque des revendications 1 à 8, ou la prodrogue selon la revendication 9, pour son utilisation pour le traitement et/ou la prévention du cancer.

## Patentansprüche

1. Verbindung der folgenden Formel (I): 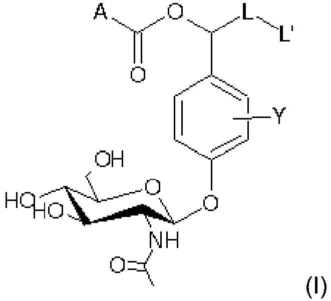 wobei:
- A ein Mittel gegen Krebs ist,
- Y eine elektrophile oder elektronenabgebende Gruppe ist,
L einen Verknüpfer entsprechend der folgenden Formel (II) darstellt:
-A--A₂-A₃-A₄-A₅-A₆- (II)
wobei:
. A₁ einen (C₁-C₆)-Alkylenrest darstellt,
. A₂ eine durch Klick-Chemie erhaltene Gruppe darstellt,
. A₃ einen (C₁-C₆)-Alkylenrest darstellt,
. A₄ einen (C₁-C₃₂)-Alkylenrest darstellt, der durch mindestens ein Sauerstoffatom unterbrochen ist und vorzugsweise ein Polyoxyalkylenrest ist,
. A₅ einen (C₁-C₆)-Alkylenrest darstellt,
. A ₆ ein Rest ist, der aus der Gruppe ausgewählt ist, bestehend aus: -NR_{c}-, -O- und -S-, wobei R_{c} H oder eine (C₁-C₁₂)-Alkylgruppe darstellt, und
L' einen Rest darstellt, der geeignet ist, um mit einer Amino-, Hydroxy- oder Thiolfunktion zu reagieren, und vorzugsweise einer Thiolfunktion,
sowie pharmazeutisch verträgliche Salze davon oder ein racemisches, diastereomeres oder enantiomeres Gemisch davon.

2. Verbindung nach Anspruch 1, wobei A₂ eine Triazolgruppe ist.

3. Verbindung nach Anspruch 1 oder 2, entsprechend der folgenden Formel (III): 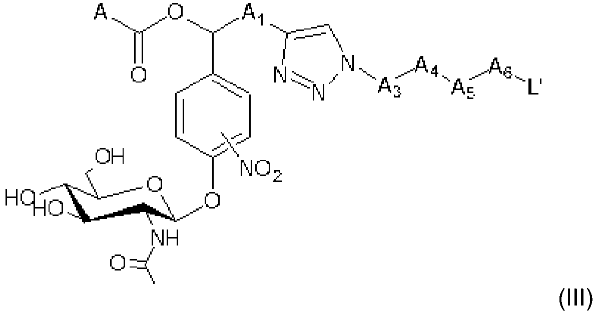 wobei A, A₁, A₃, A₄, A₅, A₆ und L' wie in Anspruch 1 definiert sind.

4. Verbindung nach einem der Ansprüche 1 bis 3, wobei A₄ eine Gruppe der Formel -(CH₂-O-CH₂)ₙ- ist, wobei n eine ganze Zahl von 1 bis 12 ist.

5. Verbindung nach einem der Ansprüche 1 bis 4, die der folgenden Formel (IV) entspricht: 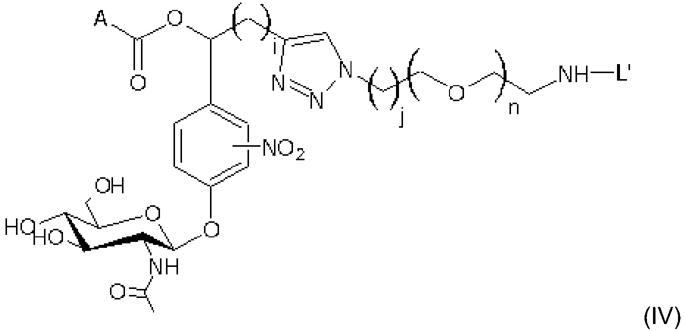
wobei i eine ganze Zahl von 1 bis 6 ist,
j eine ganze Zahl von 1 bis 6 ist,
n eine ganze Zahl von 1 bis 12 ist, und
A und L' wie in Anspruch 1 definiert sind.

6. Verbindung nach einem der Ansprüche 1 bis 5, wobei L' eine Maleimidocaproylgruppe ist.

7. Verbindung nach einem der Ansprüche 1 bis 6, wobei A Monomethylauristatin E, Doxorubicin oder ein Derivat davon ist.

8. Verbindung nach einem der Ansprüche 1 bis 7, die der folgenden Formel entspricht:

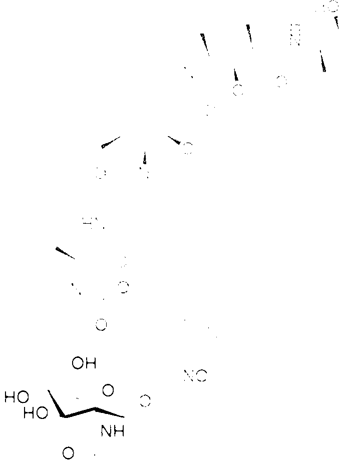

9. Prodrug, umfassend die Verbindung nach einem der Ansprüche 1 bis 8, die durch eine kovalente Bindung an ein Albuminmolekül oder eines seiner Fragmente oder Derivate gebunden ist.

10. Verbindung nach einem der Ansprüche 1 bis 8 oder Prodrug nach Anspruch 9 für seine Verwendung als Arzneimittel.

11. Pharmazeutische Zusammensetzung, umfassend eine Verbindung nach einem der Ansprüche 1 bis 8 oder Prodrug nach Anspruch 9 oder ein pharmazeutisch verträgliches Salz sowie mindestens einen pharmazeutisch verträglichen Hilfsstoff.

12. Verbindung nach einem der Ansprüche 1 bis 8 oder Prodrug nach Anspruch 9 für seine Verwendung bei der Behandlung und/oder der Verhinderung von Krebs.

## Claims

1. Compound of the following formula (I): 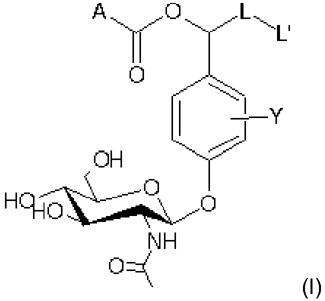 wherein:
- A is an anticancer agent,
- Y is an electron-withdrawing or electron-donating group,
- L represents a linker corresponding to the following formula (II):
-A- -A₂-A₃-A₄-A₅-A₆- (II)
wherein:
. A₁ represents a (C₁ -C₆ )alkylene radical,
. A₂ represents a group obtained by click chemistry,
. A₃ represents a (C₁ -C₆ )alkylene radical,
. A₄ represents a (C₁ -C₃₂ )alkylene radical interrupted by at least one oxygen atom, and preferably being a polyoxyalkylene radical,
. A₅ represents a (C₁ -C₆ )alkylene radical,
. A₆ is a radical selected from the group consisting of: -NR_{c} -, -O- and -S-, R_{c} representing H or a (C₁ -C₁₂ )alkyl group, and
- L represents a radical capable of reacting with an amino, hydroxy or thiol function, and preferably a thiol function,
as well as the pharmaceutically acceptable salts thereof, or a racemic, diastereoisomeric or enantiomeric mixture thereof.

2. Compound according to claim 1, wherein A₂ is a triazole group.

3. Compound according to claim 1 or 2, corresponding to the following formula (III): 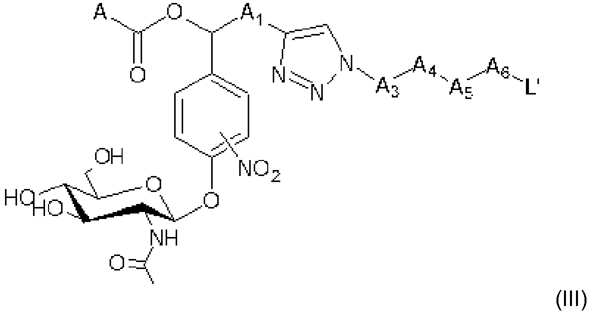 A, A₁ , A₃ , A₄ , A₅ , A₆ and L are as defined in claim 1.

4. A compound according to any of claims 1 to 3, wherein A₄ is a group of the formula -(CH₂ -O-CH₂ )ₙ -, n being an integer of 1 to 12.

5. Compound according to any of claims 1 to 4, corresponding to the following formula (IV): 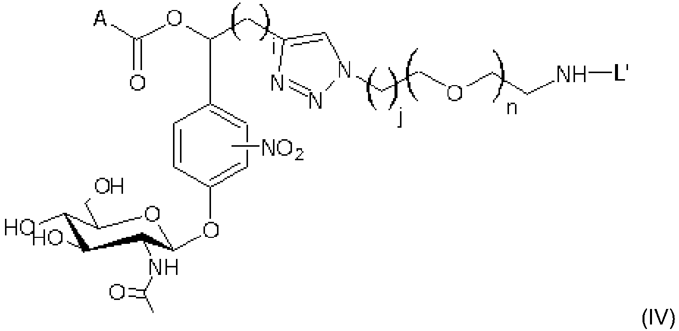
where i is an integer between 1 and 6,
j is an integer between 1 and 6,
n being an integer between 1 and 12, and
A and L' are as defined in claim 1.

6. A compound according to any of claims 1 to 5, wherein L' is a maleimidocaproyl group.

7. A compound according to any of claims 1 to 6, wherein A is monomethyl auristatin E, doxorubicin or a derivative thereof.

8. A compound according to any of claims 1 to 7, having the following formula:

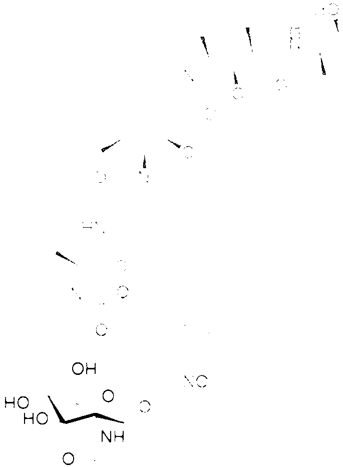

9. A prodrug comprising the compound according to any of claims 1 to 8 linked by a covalent bond to an albumin molecule or a fragment or derivative thereof.

10. A compound according to any of claims 1 to 8, or the prodrug according to claim 9, for use as a medicament.

11. Pharmaceutical composition comprising a compound according to any of claims 1 to 8 or a prodrug according to claim 9, or a pharmaceutically acceptab e or salt thereof, together with at least one pharmaceutically acceptable excipient.

12. A compound according to any of claims 1 to 8, or the prodrug according to claim 9, for use in the treatment and/or prevention of cancer.
